# EUROPÄISCHE PATENTANMELDUNG

(11) **EP 1 550 648 A1**
(43) Veröffentlichungstag der Anmeldung: **06.07.2005**
(21) Anmeldenummer: 04025482.3
(22) Anmeldetag: 27.10.2004
(51) Int. Cl.: C07C 209/26, C07C 211/19, C07C 45/50, C07C 47/347, C07C 47/445

(54) **Verfahren zur Herstellung von TCD-Diamin**

(30) Priorität: 08.11.2003 DE 10352258
(71) Anmelder: Celanese Chemicals Europe GmbH, 61476 Kronberg (DE)
(72) Erfinder: Lappe, Peter, Dr., 46539 Dinslaken (DE); Springer, Helmut, 46539 Dinslaken (DE); Lukas, Rainer, Dr., 45133 Essen (DE)

(57) **Zusammenfassung**

Die vorliegende Erfindung betrifft ein Verfahren zur Herstellung von 3(4),8(9)-Bis(aminomethyl)-tricyclo[5.2.1.0^{2.6}]decan durch Hydroformylierung von Dicyclopentadien mit nachfolgender reduktiver Aminierung. Die Hydroformylierung von Dicyclopentadien wird in zwei Stufen durchgeführt, wobei in einer ersten Hydroformylierungsstufe die Umsetzung in einem heterogenen Reaktionssystem unter Verwendung einer wässrigen Lösung, wasserlösliche organische Phosphor(III)verbindungen in komplexer Bindung enthaltender Übergangsmetallverbindungen der Gruppe VIII des Periodensystems zum 8(9)-Formyl-tricyclo[5.2.1.0^{2.6}]dec-3-en erfolgt und wobei in einer zweiten Hydroformylierungsstufe das so erhaltene 8(9)-Formyl-tricyclo[5.2.1.0^{2.6}]dec-3-en in homogener organischer Phase in Gegenwart von Übergangsmetallverbindungen der Gruppe VIII des Periodensystems der Elemente in 3(4),8(9)-Bisformyl-tricyclo[5.2.1.0^{2.6}]decan überführt wird.

## Beschreibung

Die vorliegende Erfindung betrifft ein Verfahren zur Herstellung von TCD-Diamin {3(4), 8(9)-Bis(aminomethyl)-tricyclo[5.2.1.0^{2.6}]decan} aus Dicyclopentadien (DCP).

Das durch Dimerisierung von Cyclopentadien leicht zugängliche und auch großtechnisch hergestellte Dicyclopentadien (DCP) lässt sich zu anwendungstechnisch wichtigen Verbindungen umsetzen, denen das Tricyclodecan-Gerüst besondere Eigenschaften verleiht. Die vom DCP-abgeleiteten Verbindungen mit Tricyclodecan-Struktur werden in der Literatur häufig unterschiedlich bezeichnet. In Anlehnung an die aus Chemiker-Zeitung, 98, 1974, Seiten 70 bis 76 bekannte Nomenklatur für DCP-Derivate, wird auch im folgenden die auf dem Tricyclodecan-Gerüst, auch TCD-Gerüst genannt, aufbauende Nomenklatur verwendet.

Insbesondere die Hydroformylierung von DCP liefert interessante TCD-Aldehyde, wie 8(9)-Formyl-tricyclo[5.2.1.0^{2.6}]dec-3-en, auch als TCD-Monenal bezeichnet, oder 3(4),8(9)-Bisformyl-tricyclo[5.2.1.0^{2.6}]decan, auch als TCD-Dialdehyd bezeichnet, die zu wichtigen Zwischenprodukten weiterverarbeitet werden. Wegen ihrer thermischen Labilität, die bei der Destillation zu Verlusten führt, werden die TCD-Aldehyde zumeist nicht rein isoliert, sondern als Rohprodukte der Hydroformylierungsreaktion weiterverarbeitet. So führt die reduktive Aminierung von TCD-Dialdehyd zu TCD-Diamin {3(4),8(9)-Bis(aminomethyl)-tricyclo[5.2.1.0^{2.6}]decan}, das als wertvolles Zwischenprodukt in zahlreichen technisch ausgeübten Synthesen Verwendung findet, beispielsweise zur Herstellung von lichtbeständigen Polyurethansystemen (DE 28 19 980), zur Herstellung von zahntechnischen Materialien (WO 2002 013 767, EP 678 533), zur Synthese von Polyamiden (EP 168 816), polyfunktionellen Epoxidharzsystemen (JP 58 135 875), hitzehärtbaren Beschichtungsmaterialien (EP 59 962), von Epoxidharzhärtern (JP 54 004 992) und zur Herstellung von TCD-Diisocyanat (NL 66 14 717).

Die Herstellung von Aldehyden durch katalytische Anlagerung von Kohlenmonoxid und Wasserstoff an olefinische Doppelbindungen ist bekannt. Während diese Umsetzung früher nahezu ausschließlich mit Co als Katalysator durchgeführt wurde, arbeiten moderne Verfahren mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄₋ₓLₓ] wiedergeben lassen, wobei L einen Liganden bezeichnet und x gleich 0 oder eine ganze Zahl von 1 bis 3 ist.

Einen Sonderfall stellt die Hydroformylierung von Dienen dar. Während bei der Hydroformylierung konjugierter Diene unter den üblichen Bedingungen der Oxo-Synthese fast ausschließlich Monoaldehyde erhalten werden, lassen sich aus Dicyclopentadien (DCP) mit seinen isolierten Doppelbindungen neben den Mono- auch die Disubstitutionsprodukte gewinnen. Wegen der Gefahr der Retro-Diels-Alder-Reaktion bei den Temperaturen der Oxo-Synthese und der damit verbundenen Freisetzung von Cyclopentadien, das zur Komplexbildung gegenüber Übergangsmetallen befähigt ist und das die Aktivität der eingesetzten Katalysatoren mindern kann, muss die Hydroformylierung unter besonderen Bedingungen ablaufen. Es erwies sich als vorteilhaft, den früher üblichen Co-Katalysator durch Rhodium zu ersetzen, das eine hohe Selektivität der Umsetzung zu Aldehyden erreichen lässt und die Hydroformylierung bei Bedingungen erlaubt, bei denen das Ausmaß der Retro-Diels-Alder-Spaltung geringer ist. Eine zusammenfassende Darstellung über die Hydroformylierung von Dicyclopentadien und über die Weiterverarbeitung der TCD-Aldehyde findet sich in Chemiker-Zeitung 98, 1974, 70 - 76.

Die Herstellung von TCD-Diamin ist aus EP-A2-0 348 832 bekannt. Danach wird Dicyclopentadien in organischer Lösung in Gegenwart von Rhodium zum rohen TCD-Dialdehyd hydroformyliert, das anschließend ohne weitere Abtrennung des TCD-Dialdehyds und ohne Entfernung des Katalysators in einem zweiten Reaktionsschritt reduktiv aminiert wird. Nach der bekannten Arbeitsweise umgeht man die Probleme, die mit der schwierigen Abtrennung des reaktiven TCD-Dialdehyds aus dem Hydroformylierungsgemisch verbunden sind. Die thermische Behandlung des sehr reaktiven TCD-Dialdehyds während der destillativen Abtrennung führt nämlich zu erheblichen Produktverlusten.

Nach der JP 10 087 573 erfolgt die Herstellung des TCD-Diamins durch Hydrierung des entsprechenden TCD-Dialdehyddioxims in Gegenwart von Fe-Cr-modifizierten Ra-Nickel.

Die bekannten Verfahren ermöglichen die Herstellung von TCD-Diamin in nur wirtschaftlich mäßigen Selektivitäten und Ausbeuten. Daher besteht ein Bedarf nach einem möglichst einfachen und kostengünstigen Verfahren zur Hydroformylierung von DCP mit nachfolgender Herstellung von TCD-Diamin.

Die Erfindung besteht daher in einem Verfahren zur Herstellung von 3(4),8(9)-Bis(aminomethyl)-tricyclo[5.2.1.0^{2.6}]decan durch Hydroformylierung von Dicyclopentadien mit nachfolgender reduktiver Aminierung. Es ist dadurch gekennzeichnet, dass man Dicyclopentadien in einer ersten Hydroformylierungsstufe in einem heterogenen Reaktionssystem unter Verwendung einer wässrigen Lösung, wasserlösliche organische Phosphor(III)verbindungen in komplexer Bindung enthaltender Übergangsmetallverbindungen der Gruppe VIII des Periodensystems der Elemente bei Temperaturen von 70 bis 150°C und Drücken von 0,5 bis 10 MPa mit Synthesegas zum 8(9)-Formyl-tricyclo[5.2.1.0^{2.6}]dec-3-en umgesetzt, danach die organische Phase von der wässrigen Phase trennt und anschließend das so erhaltene 8(9)-Formyl-tricyclo[5.2.1.0^{2.6}]dec-3-en in einer zweiten Hydroformylierungsstufe in homogener organischer Phase in Gegenwart von Übergangsmetallverbindungen der Gruppe VIII des Periodensystems der Elemente bei Temperaturen von 70 bis 140°C und Drücken von 5 bis 35 MPa durch Umsetzung mit Synthesegas in 3(4),8(9)-Bisformyl-tricyclo[5.2.1.0^{2.6}]-decan überführt und das so erhaltene 3(4),8(9)-Bisformyl-tricyclo[5.2.1.0^{2.6}]-decan anschließend zum 3(4),8(9)-Bis(aminomethyl)-tricyclo[5.2.1.0^{2.6}]-decan reduktiv aminiert.

Charakteristisch für das erfindungsgemäße Hydroformylierungsverfahren von Dicyclopentadien ist die zweistufige Reaktionsführung, wobei in der ersten Stufe nach dem heterogenen Zweiphasenprozess in Gegenwart einer wässrigen Katalysatorlösung gearbeitet wird und das Reaktionsprodukt der ersten Stufe, enthaltend überwiegend TCD-Monoaldehyd und geringe Mengen an nicht umgesetzten DCP, ohne weitere Reinigung in einer zweiten Stufe nach Katalysatorzusatz in einem homogenen Reaktionsmedium zum TCD-Dialdehyd umgesetzt wird, das dann anschließend zum TCD-Diamin reduktiv aminiert wird. Durch diese Art der Reaktionsführung erfolgt in der ersten Reaktionsstufe eine sehr selektive Hydroformylierung der im Sechsring des TCD-Gerüstes vorhandenen Doppelbindung zu TCD-Monoaldehyd, der häufig auch als TCD-Monenal {8(9}-Formyl-tricyclo[5.2.1.0^{2.6}]dec-3-en} bezeichnet wird.

Überraschenderweise zeigt sich, dass das Reaktionsprodukt der ersten Hydroformylierungsstufe nach Abtrennung der wässrigen Katalysatorphase ohne weitere Reinigung in einem homogenen organischen Medium nach Katalysatorzusatz zum TCD-Dialdehyd hydroformyliert werden kann, obwohl die das Wertprodukt enthaltende organische Phase homogen gelöste und analytisch nachweisbare Mengen an Phosphor- und Schwefel-Spalt- und Abbauprodukten enthält, die als Katalysatorgifte in der Oxo-Synthese bekannt sind.

Nach "New Synthesis with Carbon Monoxide" (Edited by J. Falbe, Springer-Verlag 1980, Reactivity and Structure Concepts in Organic Chemistry, Vol. 11, Seite 73) sind bei der rhodiumkatalysierten Hydroformylierung zahlreiche Katalysatorgifte bekannt. Neben Halogen, Acetylenen und Carbonsäuren wird vor allem auch auf Schwefel hingewiesen. Bereits geringe Mengen dieser Katalysatogifte bewirken eine drastische Deaktivierung des Hydroformylierungskatalysators.

Auch für den nachfolgenden Reaktionsschritt kann das TCD-Dialdehyd haltige Rohprodukt ohne zwischengeschaltete Reinigungsschritte, wie zum Beispiel Destillations- oder Waschschritte, eingesetzt werden. Die reduktive Aminierung des TCD-Dialdehyds zum TCD-Diamin erfolgt nach konventionellen Verfahren.

Dies ist insofem überraschend, weil auch bei den in der Technik durchgeführten Hydrierverfahren an Festbettkatalysatoren der Einfluß von Katalysatorgiften in zahlreichen Publikationen beschrieben wird. In Ullmann's Encyclopedia of Industrial Chemistry, 5. Auflage, 1985, Vol. A1, Seite 283, wird auf die Wirkung von schwefelhaltigen Katalysatorgiften bei heterogenkatalysierten Hydrierverfahren hingewiesen.

Ferner weist die DOS 29 18 107 vor einer Weiterverarbeitung von TCD-Monenal {(8)9-Formyl-tricyclo[5.2.1.0^{2.6}]dec-3-en} zum entsprechenden gesättigten Aldehyd TCD-Monal auf eine destillative Reinigung des ungesättigten Aldehyds hin.

Auch die Lehre in DOS 26 54 221 deutet darauf hin, für den nachfolgenden Hydrierschritt destillativ aufgearbeitetes TCD-Monenal einzusetzen.

Überraschenderweise zeigte sich ebenfalls, dass das in der ersten Stufe nicht vollständig umgesetzte Dicyclopentadien in der zweiten Hydroformylierungsstufe ohne Bildung von hochsiedenden Nebenprodukten vollständig in den TCD-Dialdehyd überführt werden kann. Daraus ergibt sich die vorteilhafte Möglichkeit einer DCP-Teilumsatzfahrweise in der ersten Hydroformylierungsstufe.

Eine destillative Reinigung von TCD-Monenal aus der abgetrennten organischen Phase der ersten Hydroformylierungsstufe ist jedoch nicht ausgeschlossen. Diese Arbeitsweise bedarf jedoch eines zusätzlichen Destillationsschrittes und führt, wenn auch zu nur geringen Destillationsverlusten. Die gezielte Herstellung von TCD-Monenal aus Dicyclopentadien unter Verwendung einer wässrigen Katalysatorlösung sowie die destillative Reinigung ist aus EP-B1-0 186 075 bekannt.

Die erste Reaktionsstufe des neuen Verfahrens führt man als heterogene Reaktion im einem Zweiphasensystem durch, eine Umsetzung, die z.B. in der DE-B-26 27 354 beschrieben ist. Dieser Prozess ist charakterisiert durch das Vorliegen einer organischen Phase, die das olefinische Ausgangsmaterial und das Reaktionsprodukt enthält, und einer wässrigen Phase, in der der Katalysator gelöst ist. Als Katalysatoren werden wasserlösliche Rhodium-Komplexverbindungen eingesetzt, die wasserlösliche organischen Phosphor(III)-Verbindungen als Liganden enthalten. Beispiele für wasserlösliche Phosphor(III)-Verbindungen, die mit Rhodium Komplexverbindungen bilden, sind Triarylphosphine, Trialkylphosphine, gemischte aliphatische-aromatische Phosphine und arylierte bzw. alkylierte Diphosphine, deren organische Reste Sulfonsäuregruppen oder Carboxylgruppen enthalten. Ihre Herstellung und Anwendung ist z.B. aus DE-B 26 27 354, EP-B1-0 103 810, EP-B1-0 163 234 und EP-A1-0 571 819 bekannt. Weitere Gruppen geeigneter Verbindungen sind sulfonierte oder carboxylierte organische Phosphite sowie heterocyclische Verbindungen des dreiwertigen Phosphors, die z.B. aus EP-A1-0 575 785 und EP-A1-0 646 588 bekannt sind.

Als sulfonierte Arylphosphine eignen sich in dem erfindungsgemäßen Verfahren sulfonierte Triarylphosphine der allgemeinen Formel (I) 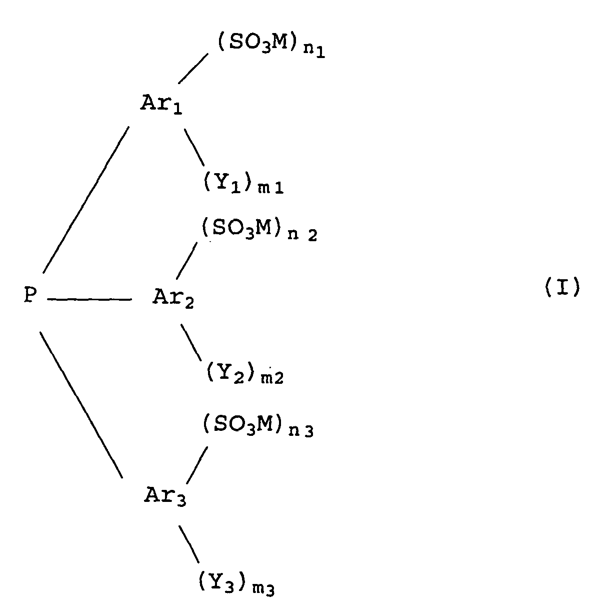 in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

Zu den Triarylphosphinen zählen bevorzugt solche Triarylphosphine, bei denen die Gruppen Ar¹, Ar², Ar³ Phenylgruppen sind; Y₁, Y₂ und Y₃ für die Methyl-, die Ethylgruppe, für die Methoxy-, Ethoxygruppe und/oder für ein Chloratom stehen; und die kationischen Reste M anorganische Kationen von Natrium, Kalium, Calcium und Barium sind. Insbesondere geeignet sind solche Triarylphosphine, bei denen Ar¹, Ar², Ar³ jeweils eine Phenylgruppe bedeuten, m₁, m₂, m₃ gleich 0 sind, n₁, n₂ und n₃ gleich 0 oder 1 sind und n₁+n₂+n₃ zusammen 1 bis 3 ausmachen, und bei denen die Sulfonat-Gruppen in meta-Stellung stehen.

Ein für die Durchführung des erfindungsgemäßen Hydroformylierungsverfahrens geeignetes Gemisch an (Sulfophenyl)-diphenylphosphin, Di-(sulfophenyl)phenylphosphin und Tri(sulfophenylphosphin) fällt bei der Sulfonierung von Triphenylphosphin an, wie z.B. aus DE-OS 26 27 354 bekannt. Im Stand der Technik wird (Sulfophenyl)diphenylphosphin als TPPMS, Di-(sulfophenyl)phenylphosphin als TPPDS und Tri(sulfophenyl)phosphin als TPPTS abgekürzt.

Als sulfonierte Arylphosphine eignen sich ebenfalls sulfonierte Diphosphine der allgemeinen Formeln (II) oder (III)

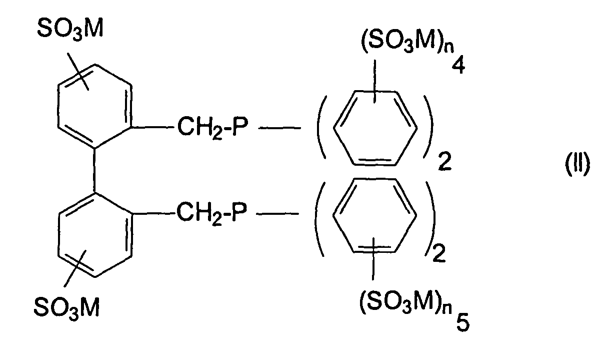

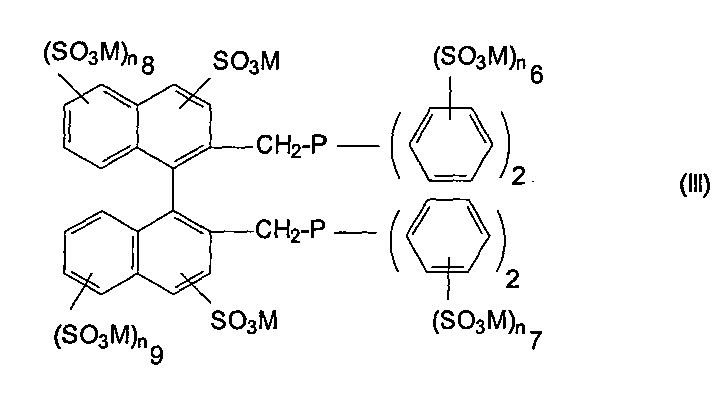

Diese Diphosphine der allgemeinen Formeln (II) und (III) sind aus W098/30526 bekannt.

In (II) steht ein jedes n₄ und n₅ unabhängig voneinander für 0 oder 1, wobei die Verbindung der Formel (II) bis sechs -SO₃M-Gruppen enthält.

In (III) steht ein jedes n₆, n₇, n₈ und n₉ unabhängig voneinander für 0 oder 1, wobei die Verbindung der Formel (III) vier bis acht -SO₃M-Gruppen enthält.

Aufgrund der Herstellung durch Sulfonierung der entsprechenden Diphosphine der Formeln (IIa) und (IIIa), die keine -SO₃M-Gruppen enthalten, 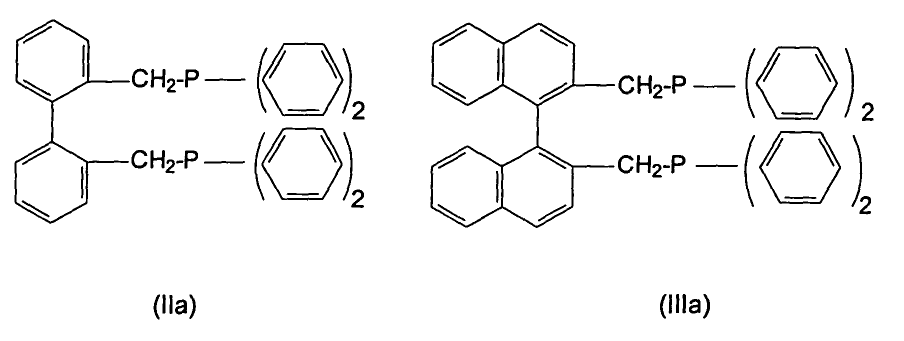 erhält man üblicherweise Gemische von Verbindungen (II) und (III) mit unterschiedlicher Anzahl von -SO₃M-Gruppen. So enthält eine Verbindung der Formeln (II) oder (III), die beispielsweise drei -SO₃M-Gruppen enthält, auch Verbindungen mit lediglich zwei -SO₃M-Gruppen aber auch Verbindungen mit vier oder fünf -SO₃M-Gruppen. Eine Verbindung der Formeln (II) oder (III) mit beispielsweise fünf -SO₃M-Gruppen enthält üblicherweise auch Verbindungen mit lediglich drei oder vier -SO₃M-Gruppen aber auch Verbindungen mit sechs oder sieben -SO₃M-Gruppen.

Verbindungen der Formel (II) besitzen maximal sechs -SO₃M-Gruppen, während Verbindungen der Formel (III) maximal acht -SO₃M-Gruppen aufweisen.

Aus diesem Grund gelangen in der Regel Mischungen von Verbindungen der Verbindungen (II) und (III) mit unterschiedlicher Anzahl von -SO₃M-Gruppen zum Einsatz.

In den Formeln (II) und (III) steht M für Ammonium, ein einwertiges Metall oder das Äquivalent eines mehrwertigen Metalls, insbesondere für Natrium, Kalium, Calcium oder Barium.

Besonders vorteilhaft ist der Einsatz wasserlöslicher Komplexverbindungen des Rhodiums, obwohl die Verwendung anderer katalytisch aktiver Übergangsmetallverbindungen der Gruppe VIII des Periodensystems der Elemente nicht ausgeschlossen wird. So kann man in der ersten Hydroformylierungsstufe auch wasserlösliche Komplexverbindungen von Kobalt, Iridium, Nickel, Palladium, Platin, Eisen oder Ruthenium verwenden, und besonders wasserlösliche Komplexverbindungen des Kobalts, Iridiums und Platins haben sich als wirksame Hydroformylierungskatalysatoren erwiesen.

Die Bedingungen, unter denen die Umsetzung in der ersten Hydroformylierungsstufe abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung der Einsatzstoffe bei Temperaturen von 70 bis 150°C durch. Bevorzugt hält man Temperaturen von 100 bis 150°C und insbesondere von 110 bis 140°C ein. Der Gesamtdruck erstreckt sich über einen Bereich von 0,5 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Die Rhodium-Konzentration beträgt 20 bis 1000 Gew.-ppm, vorzugsweise 50 bis 800 Gew.-ppm und insbesondere 100 bis 600 Gew.-ppm, jeweils bezogen auf die wässrige Katalysatorlösung. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphorliganden, d.h. Ligand, der mit Rhodium keine komplexe Bindung eingegangen ist. Je mol Rhodium wendet man bevorzugt 10 bis 300 mol Phosphor in Form einer wasserlöslichen organischen Phosphorverbindung an. Besonders bewährt haben sich molare Verhältnisse von Rhodium zu Phosphor im Bereich von 1:50 bis 1:150. Der Rhodium-Phosphor-Komplexkatalysator braucht nicht einheitlich zusammengesetzt sein, sondern kann z.B. aus einem Gemisch von Rhodium-Komplexverbindungen bestehen, die sich durch die Art der Phosphor-Liganden unterscheiden. Ebenso kann der in der wässrigen Katalysatorlösung enthaltene freie Phosphor-Ligand aus einem Gemisch unterschiedlicher wasserlöslicher organischer Phosphorverbindungen zusammengesetzt sein.

Wird ein anderes Übergangsmetall der Guppe VIII des Periodensystems der Elemente als katalytisch aktives Metall eingesetzt, so bewegt sich die Konzentration an Übergangsmetall sowie das molare Verhältnis von Übergangsmetall zu Phosphor in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Man bildet den Katalysator üblicherweise aus den Komponenten Übergangsmetall oder Übergangsmetallverbindung, organische Phosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Dicylopentadien kann als solches oder in Lösung der Hydroformylierung zugeführt werden. Geeignete Lösungsmittel sind wasserunlösliche Ketone, Dialkylether, aliphatische Nitrile, aromatische Kohlenwasserstoffe wie Benzol oder Toluol und gesättigte cycloaliphatische Kohlenwasserstoffe wie Cyclopentan oder Cylcohexan oder gesättigte aliphatische Kohlenwasserstoffe.

Um den Umsatz je Zeiteinheit von Dicyclopentadien, das in der wässrigen Katalysatorlösung nur wenig löslich sind, zu erhöhen, kann es sich empfehlen, dieser Lösung ein Phasentransferreagenz (Lösungsvermittler) zuzusetzen. Er verändert die physikalischen Eigenschaften der Grenzflächen zwischen den beiden flüssigen Phasen und erleichtert den Übergang des organischen Reaktanten in die wässrige Katalysatorlösung.

Als Lösungsvermittler sind Verbindungen bekannt, deren hydrophile Gruppen ionisch (anionisch oder kationisch) oder nicht ionisch sind. Zu den anionenaktiven Verbindungen gehören Natrium-, Kalium- oder Ammoniumsalze von Carbonsäuren, vorzugsweise solcher mit 8 bis 20 Kohlenstoffatomen und insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagenzien dissoziieren in wässriger Lösung nicht in Ionen. Zu ihnen zählen Alkylpolyethylenglykole, Alkylphenylpolyethylenglykole, Fettsäurealkylolamine und Trialkylaminoxide. Ferner finden Ampholyte wie Aminocarbonsäuren, Betaine und Sulfobetain als Lösungsvermittler Anwendung. Entsprechende Verfahren sind z.B. aus EP-B1-0 157 316 bekannt.

Es können auch Rhodiumkomplexverbindungen eingesetzt werden, die gleichzeitig Katalysator und Phasentransferreagenz sind. Eine solche Arbeitsweise ist z.B. Gegenstand der EP-B1-0 163 234.

Auch hinsichtlich der verfahrenstechnischen und apparativen Ausgestaltung der ersten Stufe des neuen Verfahrens kann man sich innerhalb weiter Grenzen bewegen. Eine bewährte Ausführungsform der heterogenen Hydroformylierung unter Verwendung einer wässrigen Katalysatorphase ist in der EP-B1-0 103 810 beschrieben. Der Reaktionsaustrag der ersten Hydroformylierungsstufe wird in einem Phasentrenner in die organische Produktphase und in die wässrige Katalysatorlösung aufgetrennt. Es hat sich als zweckmäßig erwiesen, die Katalysatorlösung im Kreis zu führen. Die rohe organische Produktphase wird ohne weitere Reinigungsschritte der zweiten Hydroformylierungsstufe zugeführt. Eine zwischengeschaltete destillative Reinigung des Reaktionsproduktes der ersten Hydroformylierungssufe kann aber gegebenenfalls auch durchgeführt werden.

Die zweite Hydroformylierungsstufe des neuen Verfahrens führt man in einem homogenen Reaktionssystem durch. Der Begriff homogenes Reaktionssystem steht für eine im wesentlichen aus Lösungsmittel, falls in der ersten Stufe und/oder in der zweiten Reaktionsstufe zugesetzt, Katalysator, unumgesetztes Dicyclopentadien und TCD-Monenal zusammengesetzte homogene Lösung. Gegebenenfalls kann sich ein Lösungsmittelzusatz in der zweiten Reaktionsstufe als zweckmäßig erweisen. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether, wie Tetrahydrofuran, Ketone oder Texanol® der Firma Eastman. Der Anteil des Lösungsmittels im Reaktionsmedium kann über einen weiten Bereich variiert werden und beträgt üblicherweise zwischen 10 und 80 Gew.-%, vorzugsweise 20 bis 50 Gew.-%, bezogen auf das Reaktionsgemisch.

Ein Lösungsmittelzusatz in der zweiten, wie auch in der ersten Hydroformylierungsstufe, ist jedoch nicht zwingend erforderlich.

Als Katalysatoren in der zweiten Hydroformylierungsstufe verwendet man Übergangsmetallverbindungen der Gruppe VIII des Periodensystems der Elemente, vorzugsweise Verbindungen des Kobalts, Rhodiums, Iridiums, Nickels, Eisens, Platins, Palladiums oder Rutheniums, und insbesondere des Kobalts, Rhodiums und Iridiums. Besonders bevorzugt ist die Verwendung von Rhodium. Dabei werden im allgemeinen Rhodium-Verbindungen verwendet, die nicht mit phosphorhaltigen Liganden, wie Phosphinen oder Phosphiten modifiziert sind. Solche, nicht mit Phosphinen oder Phosphiten modifizierten Rhodiumkatalysatoren und ihre Eignung als Katalysator zur Hydroformylierung sind aus der Literatur bekannt und sie werden als unmodifizierte Rhodiumkatalysatoren bezeichnet. Es wird in der Fachliteratur angenommen, dass die Rhodium-Verbindung HRh(CO)₄ die katalytisch aktive Rhodiumspezies bei der Hydroformylierung mit unmodifizierten Rhodiumkatalysatoren ist, obgleich dies aufgrund der vielen in der Hydroformylierungszone nebeneinander ablaufenden Chemismen nicht eindeutig bewiesen ist. Da im allgemeinen die Verwendung von nicht mit Phosphinen modifizierten Rhodiumkatalysatoren einen geringeren Rhodiumgehalt erfordert, arbeitet man vorzugsweise in der zweiten Hydroformylierungsstufe mit unmodifizierten Rhodiumkatalysatoren. Der Rhodiumgehalt beträgt im allgemeinen von 5 bis 100 ppm, bezogen auf das homogene Reaktionsgemisch.

Man kann aber auch in der zweiten Hydroformylierungsstufe Rhodium-Komplexverbindungen verwenden, die organische Phosphor(III)-Verbindungen als Liganden enthalten. Derartige Komplexverbindungen und ihre Herstellung sind bekannt (z.B. aus US-A-3 527 809, US-A-4 148 830, US-A-4 247 486, US-A-4 283 562). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von etwa 5 bis etwa 1000 Gew.-ppm und beträgt vorzugsweise 10 bis 700 Gew.-ppm. Insbesondere wendet man Rhodium in Konzentrationen von 20 bis 500 Gew.-ppm, jeweils bezogen auf das homogene Reaktionsgemisch an. Als Katalysator kann die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigen Phosphor-Liganden durchzuführen, der mit Rhodium keine Komplexverbindung mehr eingeht. Der freie Phosphor-Ligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Phosphor-Komplexverbindungen, die als Katalysatoren Anwendung finden können, sind in US-A-3 527 809 beschrieben. Zu den bevorzugten Liganden in den Rhodium-Komplexkatalysatoren zählen z. B. Triarylphosphine wie Triphenylphosphin, Trialkylphosphine wie Tri-(n-octyl)-phosphin, Trilaurylphosphin, Tri-(cyclohexyl)-phosphin, Alkylphenylphosphine, Cycloalkylphenylphosphine und organische Diphosphite. Wegen seiner leichten Zugänglichkeit wird Triphenylphosphin besonders häufig angewandt.

Arbeitet man mit einem modifizierten Rhodium-Komplexkatalysatorsystem, beträgt üblicherweise in der homogenen Reaktionsmischung das molare Verhältnis von Rhodium zu Phosphor 1 : 5 bis 1 : 200, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 10 bis 1 : 100 ein.

Verwendet man in der zweiten Hydroformylierungsstufe ein anderes Übergangsmetall der Gruppe VIII des Periodensystems der Elemente als Rhodium, so liegt die Konzentration an Übergangsmetall und das molare Verhältnis von Übergangsmetall zu Phosphor, falls man nach dem phosphinmodifizierten Verfahren arbeitet, in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Die Bedingungen, unter denen die Umsetzung in der zweiten Hydroformylierungsstufe abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die zweite Hydroformylierungsstufe des rohen TCD-Monenal's bei Temperaturen von 70 bis 140°C durch. Bevorzugt hält man Temperaturen von 80 bis 130°C und insbesondere von 90 bis 120°C ein. Der Gesamtdruck erstreckt sich über einen Bereich von 5 bis 35 MPa, vorzugsweise 10 bis 30 MPa und insbesondere 20 bis 30 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Man bildet den Katalysator üblicherweise aus den Komponenten Übergangsmetall oder Übergangsmetallverbindung und Synthesegas unter den Bedingungen der Hydroformylierungsreaktion im Reaktionsgemisch, gegebenenfalls in Gegenwart von organischen Phosphor(III)-Verbindungen. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Zur Herstellung des Hydroformylierungskatalysators für die erste und zweite Reaktionsstufe gelangt das Übergangsmetall der Gruppe VIII des Periodensystems der Elemente, insbesondere Rhodium, entweder in metallischer Form oder als Verbindung zum Einsatz. In metallischer Form verwendet man das Übergangsmetall entweder als feinverteilte Partikel oder in dünner Schicht auf einem Träger, wie Aktivkohle, Calciumcarbonat, Aluminiumsilikat, Tonerde niedergeschlagen. Als Übergangsmetallverbindungen eignen sich Salze aliphatischer Mono- und Polycarbonsäuren, wie Übergangsmetall-2-Ethylhexanoate, -Acetate, -Oxalate, -Propionate oder -Malonate. Weiterhin können Salze anorganischer Wasserstoff- und Sauerstoffsäuren, wie Nitrate oder Sulfate, die verschiedenen Übergangsmetalloxide oder auch Übergangsmetallcarbonylverbindungen, wie Rh₃(CO)₁₂, Rh₆(CO)₁₆, Co₂(CO)₈, Co₄(CO)₁₆, Fe(CO)₅, Fe₂(CO)₉, Ir₂(CO)₈, Ir₄(CO)₁₂ oder Übergangsmetall-Komplexverbindungen, z.B. Cyclopentadienylrhodiumverbindungen, Rhodiumacetylacetonat, Cyclopentadienylkobalt-Cyclooctadien-1,5, Fe(CO)₃-Cyclooctadien-1,5, [RhCl(Cyclooctadien-1,5]₂ oder PtCl₂(Cyclooctadien-1,5) eingesetzt werden. Übergangsmetallhalogenverbindungen kommen wegen ihres korrosiven Verhaltens der Halogenidionen weniger in Betracht.

Bevorzugt werden Übergangsmetalloxide und insbesondere Übergangsmetallacetate und -2-ethylhexanoate. Als besonders geeignet hat sich Rhodiumoxid, Rhodiumacetat, Rhodium-2-ethylhexanoat, Kobaltoxid, Kobaltacetat und Kobalt-2-ethylhexanoat erwiesen.

Die einzelnen Hydroformylierungsstufen können sowohl absatzweise als auch kontinuierlich durchgeführt werden.

Das Umsetzungsprodukt der zweiten Hydroformylierungsstufe wird ohne weitere Reinigung und ohne Katalysatorabtrennung reduktiv aminiert.

Unter reduktiver Aminierung versteht man die Reaktion des TCD-Dialdehyds mit Wasserstoff und Ammoniak in Gegenwart eines Hydrierkatalysators. Man kann aber auch den Dialdehyd zunächst mit einem primären Amin unter Bildung eines Diazomethins umsetzen und daraufhin das Diazomethin mit Wasserstoff und Ammoniak in Gegenwart eines Hydrierkatalysators behandeln. Auch diese Reaktion wird im Sinne der Erfindung als reduktive Aminierung bezeichnet.

Zur Bildung des Diazomethins gibt man zu dem nicht aufgearbeiteten Hydroformylierungsgemisch je mol Tricyclodecandialdehyd 2 bis 6 mol, insbesondere 3 bis 4 mol eines primären Amins. Die Reaktion zwischen den Ausgangsstoffen läuft bereits bei Raumtemperatur ab, sie kann durch Erhitzen auf 20 bis 60°C, insbesondere 30 bis 50°C beschleunigt werden. Als primäre Amine geeignet sind Amine mit 2 bis 10 Kohlenstoffatomen im Molekül. Mit besonderem Erfolg werden Amine mit 3 bis 5 Kohlenstoffatomen im Molekül, vorzugsweise n-Butylamin, eingesetzt.

Die reduktive Aminierung des Dialdehyds oder des Diazomethins wird zweckmäßig bei Temperaturen von 60 bis 150°C, vorzugsweise 80 bis 140°C durchgeführt. Der Wasserstoffdruck im Reaktionsgefäß beträgt bei der Reaktionstemperatur 2 bis 12 und insbesondere 8 bis 10 MPa.

Als Hydrierkatalysatoren werden Nickel oder Kobalt verwendet, entweder in Form von Raney-Nickel bzw. Raney-Kobalt oder auch in Form entsprechender Trägerkatalysatoren. Ein bevorzugter Katalysator enthält 50 bis 60 Gew.-% Nickel auf Kieselgur.

Ammoniak soll zweckmäßigerweise im Überschuß angewendet werden. Je mol Formylgruppe bzw. je mol Diazomethingruppe sind mindestens 2 mol Ammoniak erforderlich, vorzugsweise werden 4 bis 10 mol Ammoniak eingesetzt.

Die reduktive Aminierung des Dialdehyds oder des Diazomethins kann in Abwesenheit von Lösungsmitteln durchgeführt werden, üblicherweise wirkt das Endprodukt selbst als Lösungsmittel. Bei kleinen diskontinuierlichen Ansätzen ist es jedoch vorteilhaft, in einem Lösungsmittel zu arbeiten. Besonders gute Ergebnisse werden bei Verwendung von Tetrahydrofuran, Isobutanol, Butanol oder Isopropanol als Lösungsmittel erzielt.

Das Gemisch der isomeren Diamine wird in guten Ausbeuten erhalten. Nur ein kleiner Teil der Dialdehyde geht in höhermolekulare Kondensationsprodukte über. Sie sind im Reaktionsgemisch gelöst und stören die Produktentnahme aus dem Reaktor und eine störungsfreie Aufarbeitung der Rohprodukte nicht.

Zur Abtrennung der isomeren TCD-Diamine destilliert man das Reaktionsgemisch, vorzugsweise unter vermindertem Druck. Man erhält die Verbindungen als farblose Flüssigkeit, die unter Normaldruck bei etwa 310°C siedet. Der Hydrierkatalysator sowie das in der zweiten Hydroformylierungsstufe eingesetzte Übergangsmetall fallen im Destillationsrückstand an und werden nach bekannten Verfahren zurückgewonnen.

In den folgenden Beispielen wird das neue Verfahren näher erläutert. Selbstverständlich ist nicht beabsichtigt, die Erfindung auf diese speziellen Ausführungsformen zu beschränken.

### Beispiele

Die bei der analytischen Charakterisierung der Reaktionsprodukte verwendeten Abkürzungen haben folgende Bedeutung:

| | |
|---|---|
| DCP | Dicyclopentadien |
| TCD-Monenal | 8(9)-Formyl-tricyclo[5.2.1.0^{2.6}]dec-3-en |
| TCD-Dial | 3(4),8(9)-Bisformyl-tricyclo[5.2.10^{2.6}]decan |
| Tri-CP | Tricyclopentadien. |
| | |
| TPPTS bedeutet | Natrium-Triphenylphosphintrisulfonat |

### Herstellung von TCD-Diamin

### 1. Herstellung von TCD-Monenal

In einem 5 I-Autoklaven werden 2.119 g TPPTS-Lösung mit einem P(III)-Gehalt von 472 mmol/kg vorgelegt und mit 160,2 g Rh-Lösung (Rh-Gehalt: 6.423 mg/kg) versetzt. Danach wird eine Mischung aus 661,1 g Dicyclopentadien (technisch, DCP-Gehalt: 93,72 Gew.-%) und 283,0 g Toluol zugegeben. Das Reaktionsgemisch wird auf 135°C erwärmt und bei einem Synthesegasdruck von 2,5 MPa und einer Reaktionszeit von 6 Stunden umgesetzt.

Nach Reaktionsende wird abgekühlt und die obere, organische Phase von der wässrigen Katalysatorphase durch Phasentrennung abgetrennt. Die verbleibende Katalysatorphase wird erneut mit einem Gemisch aus Dicyclopentadien und Toluol versetzt und erneut umgesetzt. Dieser Vorgang wird insgesamt achtmal wiederholt.

Die organischen Phasen (Summe: 9.923 g) werden vereinigt und gaschromatographisch untersucht.

| GC-Analyse (in FI.-%, Flächenprozent) | |
|---|---|
| Vorlaufkomponenten | 0,32 |
| Toluol | 29,45 |
| DCP | 4,55 |
| TCD-Monenal | 61,30 |
| TCD-Dial | 0,81 |
| Tri-CP | 0,42 |
| Sonstige | 3,15 |

### 2. Herstellung von TCD-Dialdehyd

400 g rohes TCD-Monenal aus der ersten Reaktionsstufe werden ohne Anwendung weiterer Reinigungsschritte durch Zugabe einer toluolischen Lösung von Rh-2-ethylhexanoat auf einen Rhodiumgehalt von 20 ppm, bezogen auf die gesamte Reaktionslösung, eingestellt und in einem 1-I Autoklav vorgelegt. Das Reaktionsgemisch wird auf 120°C erwärmt und bei einem Druck von 26,0 MPa und einer Reaktionszeit von 6 Stunden umgesetzt. Nach Reaktionsende wird abgekühlt und entspannt, das erhaltene Reaktionsprodukt (455,9 g) wird gaschromatographisch untersucht.

| GC-Analyse (in FI.-%) | |
|---|---|
| Vorlaufkomponenten | 1,30 |
| Toluol | 31,70 |
| TCD-Monenal | 2,32 |
| TCD-Dial | 62,36 |
| Sonstige | 2,32 |

### 3. Herstellung von TCD-Diamin

Der nach der zweiten Hydroformylierungsstufe erhaltene TCD-Dialdehyd wird ohne weitere Reinigung in die reduktive Aminierung eingesetzt. Hierzu werden 242,0 g n-Butylamin in einem 1-I Dreihalskolben mit Rührer vorgelegt. 400,0 g TCD-Dialdehyd werden innerhalb von 90 Minuten bei einer Temperatur von 42 - 45°C zugetropft. Das Gemisch wird noch für zwei weitere Stunden bei 40 - 45°C gerührt, danach lässt man abkühlen und trennt die angefallene Wasserphase (62,2 g) von der organischen Phase (579,8 g) (Schiffsche Base). 100,0 g Toluol und 45,0 g Ni 52/35 - Katalysator der Firma Johnson-Matthey Plc - werden danach in einem 2-I Autoklav vorgelegt, zu dieser Mischung werden 550,0 g Ammoniak zugepumpt und auf 50°C aufgeheizt. Anschließend wird die aus der Umsetzung zwischen TCD-Dialdehyd und n-Butylamin zuvor gebildete Schiffsche Base innerhalb von 2 Stunden zugepumpt und das Reaktionsgemisch unter Wasserstoff auf 120°C erwärmt und bei einem Druck von 10,0 MPa und einer Reaktionszeit von 6 Stunden umgesetzt. Nach Reaktionsende wird abgekühlt, entspannt und vom Katalysator abfiltriert. Das so erhaltene Reaktionsprodukt (736,2 g) wird gaschromatographisch untersucht.

| GC-Analyse (in FI.-%) | |
|---|---|
| Vorlaufkomponenten | 0,28 |
| n-Butylamin | 26,08 |
| Toluol / Methylcylcohexan | 38,80 |
| TCD-Monoamin | 1,92 |
| TCD-Diamin | 31,52 |
| Sonstige | 1,40 |

Zur Aufarbeitung wird das rohe TCD-Diamin (700,0 g) an einer Claisenbrücke destilliert. Man erhält 250,6 g Hauptfraktion in einem Siedebereich von 115 - 129°C bei einem Druck von 3 hPa mit folgender Zusammensetzung.

| GC-Analyse (in FI.-%) | |
|---|---|
| Vorlaufkomponenten | 0,11 |
| TCD-Monoamin | 4,02 |
| TCD-Diamin | 95,46 |
| Sonstige | 0,41 |

Die Gesamtausbeute an TCD-Diamin beträgt über alle Stufen 86,8 % d.Th., bezogen auf Dicyclopentadien. TCD-Monoamin bedeutet das monofunktionelle Amin 8(9)-Aminomethyl-tricyclo[5.2.1.0^{2.6}]decan.

Das erfindungsgemäße Verfahren eröffnet einen eleganten Herstellungsweg für TCD-Diamin in hohen Ausbeuten, wobei auf die aufwendige Reinigung der Zwischenstufen verzichtet werden kann.

## Patentansprüche

1. Verfahren zur Herstellung von 3(4),8(9)-Bis(aminomethyl)-tricyclo[5.2.1.0^{2.6}]decan durch Hydroformylierung von Dicyclopentadien mit nachfolgender reduktiver Aminierung, **dadurch gekennzeichnet, dass** man Dicyclopentadien in einer ersten Hydroformylierungsstufe in einem heterogenen Reaktionssystem unter Verwendung einer wässrigen Lösung, wasserlösliche organische Phosphor(III)verbindungen in komplexer Bindung enthaltender Übergangsmetallverbindungen der Gruppe VIII des Periodensystems der Elemente bei Temperaturen von 70 bis 150°C und Drücken von 0,5 bis 10 MPa mit Synthesegas zum 8(9)-Formyl-tricyclo[5.2.1.0^{2.6}]dec-3-en umgesetzt, danach die organische Phase von der wässrigen Phase trennt und anschließend das so erhaltene 8(9)-Formyl-tricyclo[5.2.1.0^{2.6}]dec-3-en in einer zweiten Hydroformylierungsstufe in homogener organischer Phase in Gegenwart von Übergangsmetallverbindungen der Gruppe VIII des Periodensystems der Elemente bei Temperaturen von 70 bis 140°C und Drücken von 5 bis 35 MPa durch Umsetzung mit Synthesegas in 3(4),8(9)-Bisformyl-tricyclo[5.2.1.0^{2.6}]decan überführt und das so erhaltene 3(4),8(9)-Bisformyl-tricyclo[5.2.1.0^{2.6}]decan anschließend zum 3(4),8(9)-Bis(aminomethyl)-tricyclo[5.2.1.0^{2.6}]decan reduktiv aminiert.

2. Verfahren gemäß Anspruch 1, **dadurch gekennzeichnet, dass** das in der ersten Hydroformylierungsstufe erhaltene 8(9)-Formyl-tricyclo[5.2.1.0^{2.6}]dec-3-en vor dem Einsatz in die zweite Hydroformylierungsstufe destilliert wird.

3. Verfahren gemäß Anspruch 1 oder 2, **dadurch gekennzeichnet, dass** in der zweiten Hydroformylierungsstufe die Umsetzung in Gegenwart von organischen Phosphor(III)-Verbindungen erfolgt.

4. Verfahren gemäß Anspruch 3, **dadurch gekennzeichnet, dass** man als organische Phosphor(III)-Verbindungen Triarylphosphine, Trialkylphosphine, Alkylphenylphosphine, Cycloalkylphenylphosphine und organische Diphosphite verwendet.

5. Verfahren gemäß einem oder mehreren der Ansprüch 1 bis 4, **dadurch gekennzeichnet, dass** in der ersten Hydroformylierungsstufe als wasserlösliche organische Phoshor(III)-Verbindungen sulfonierte Triarylphosphine der allgemeinen Formel (I) 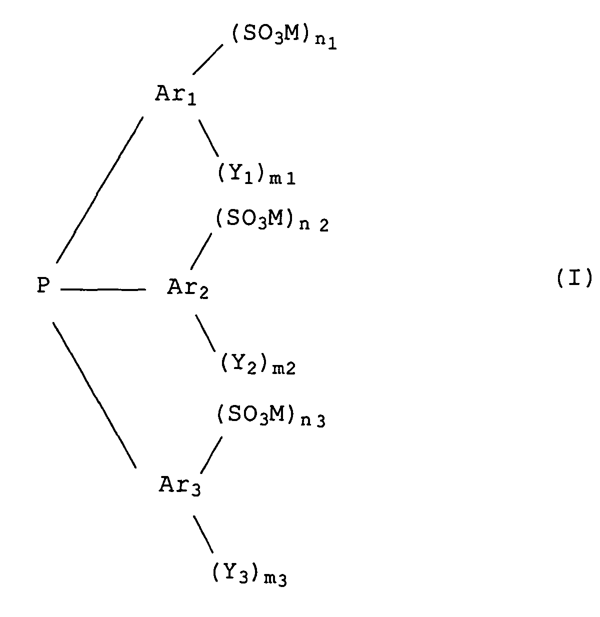 eingesetzt werden, in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

6. Verfahren gemäß Anspruch 5, **dadurch gekennzeichnet, dass** Ar₁, Ar₂, Ar₃ jeweils eine Phenylgruppe bedeuten, m₁, m₂, m₃ gleich 0 sind, n₁, n₂, n₃ gleich 0 oder 1 sind und n₁+n₂+n₃ zusammen 1 bis 3 ausmachen, und dass die Sulfonat-Gruppen in meta-Stellung stehen.

7. Verfahren gemäß einem oder mehreren der Ansprüche 1 bis 4, **dadurch gekennzeichnet, dass** in der ersten Hydroformylierungsstufe als wasserlösliche organische Phosphor(III)-Verbindungen sulfonierte Diphosphine der allgemeinen Formel (II) 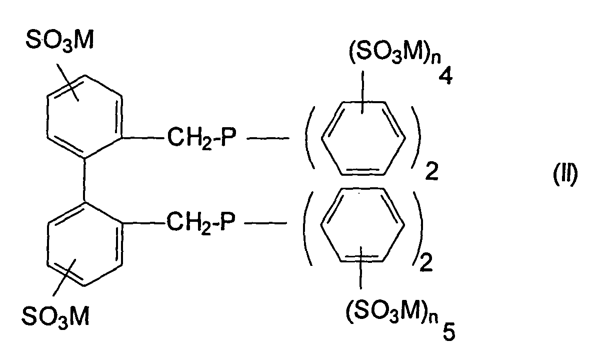 in der n₄ und n₅ unabhängig voneinander für 0 oder 1 steht, wobei die sulfonierten Diphosphine der allgemeinen Formel (II) bis zu sechs SO₃M-Gruppen enthalten, und M Ammonium, ein einwertiges Metall oder das Äquivalent eines mehrwertigen Metalls bedeutet.

8. Verfahren gemäß einem oder mehreren der Ansprüche 1 bis 4, **dadurch gekennzeichnet, dass** in der ersten Hydroformylierungsstufe als wasserlösliche organische Phosphor(III)-Verbindungen sulfonierte Diphosphine der allgemeinen Formel (III) 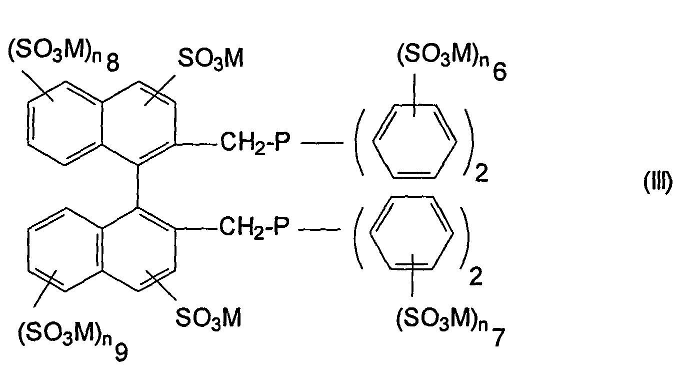 in der n₆, n₇, n₈ und n₉ unabhängig voneinander für 0 oder 1 steht, wobei die sulfonierten Diphosphine der allgemeinen Formel (III) vier bis acht SO₃M-Gruppen enthalten, und M Ammonium, ein einwertiges Metall oder das Äquivalent eines mehrwertigen Metalls bedeutet.

9. Verfahren gemäß einem oder mehreren der Ansprüche 1 bis 8, **dadurch gekennzeichnet, dass** in der ersten Hydroformylierungsstufe als Übergangsmetallverbindungen der Gruppe VIII des Periodensystems der Elemente Verbindungen von Rhodium, Kobalt, Iridium, Nickel, Palladium, Platin, Eisen oder Ruthenium verwendet werden.

10. Verfahren gemäß einem oder mehreren der Ansprüche 1 bis 8, **dadurch gekennzeichnet, dass** in der zweiten Hydroformylierungsstufe als Übergangsmetallverbindungen der Gruppe VIII des Periodensystems der Elemente Verbindungen von Rhodium, Kobalt, Iridium, Nickel, Platin, Palladium, Eisen oder Ruthenium verwendet werden.

11. Verfahren gemäß einem oder mehreren der Ansprüche 1 bis 10, **dadurch gekennzeichnet**, das in der ersten und zweiten Hydroformylierungsstufe als Übergangsmetallverbindungen der Gruppe VIII des Periodensystems der Elemente Verbindungen von Rhodium eingesetzt werden.

12. Verfahren gemäß einem oder mehreren der Ansprüche 1 bis 11, **dadurch gekennzeichnet, dass** in der ersten Hydroformylierungsstufe die Temperatur 100 bis 150°C, bevorzugt 110 bis 140°C, und der Druck 1 bis 6 MPa, bevorzugt 1,5 bis 5 MPa, beträgt.

13. Verfahren gemäß einem oder mehreren der Ansprüche 1 bis 12, **dadurch gekennzeichnet, dass** in der zweiten Hydroformylierungsstufe die Temperatur 80 bis 130°C, bevorzugt 90 bis 120°C, und der Druck 10 bis 30 MPa, bevorzugt 20 bis 30 MPa beträgt.

14. Verfahren gemäß einem oder mehreren der Ansprüche 1 bis 13, **dadurch gekennzeichnet, dass** in der ersten Hydroformylierungsstufe die Rhodium-Konzentration 20 bis 1000 Gew.-ppm, vorzugsweise 50 bis 800 Gew.-ppm und insbesondere 100 bis 600 Gew.-ppm, jeweils bezogen auf die wässrige Katalysatorlösung, beträgt.

15. Verfahren zur Herstellung gemäß einem oder mehreren der Ansprüche 1 bis 14, **dadurch gekennzeichnet, dass** in der ersten Hydroformylierungsstufe je mol Rhodium 10 bis 300 mol, insbesondere 50 bis 150 mol, Phosphor in Form der wasserlöslichen organischen Phosphorverbindung verwendet werden.

16. Verfahren gemäß einem oder mehreren der Ansprüche 1, 2, 5 bis 15, **dadurch gekennzeichnet, dass** in der zweiten Hydroformylierungsstufe die Rhodium-Konzentration 5 bis 100 Gew.-ppm, bezogen auf das homogene Reaktionsgemisch, beträgt.

17. Verfahren gemäß einem oder mehreren der Ansprüche 3 bis 15, **dadurch gekennzeichnet, dass** in der zweiten Hydroformylierungsstufe die Rhodium-Konzentration 5 bis 1000 Gew.-ppm, vorzugsweise 10 bis 700 Gew.-ppm und insbesondere 20 bis 500 Gew.-ppm, jeweils bezogen auf das homogene Reaktionsgemisch, beträgt und je mol Rhodium 5 bis 200 mol, vorzugsweise 10 bis 100 mol Phosphor in Form organischer Phosphorverbindungen verwendet werden.
